# EUROPÄISCHE PATENTSCHRIFT

(11) **EP 3 417 900 B1**
(45) Veröffentlichungstag und Bekanntmachung des Hinweises auf die Patenterteilung: **19.08.2020**
(21) Anmeldenummer: 18000529.0
(22) Anmeldetag: 15.06.2018
(51) Int. Cl.: A61M 16/06

(54) **ATEMMASKE MIT MITTELN ZUR ANKOPPLUNG DER BÄNDERUNG**
RESPIRATORY MASK WITH MEANS FOR COUPLING THE HARNESS
MASQUE RESPIRATOIRE DOTÉ DES MOYENS PERMETTANT DE COUPLER L'AGENCEMENT DE BRIDES

(30) Priorität: 19.06.2017 DE 102017005693; 19.06.2017 DE 102017005704
(43) Veröffentlichungstag der Anmeldung: 26.12.2018
(73) Patentinhaber: Löwenstein Medical Technology S.A., 2557 Luxembourg (LU)
(72) Erfinder: Frerichs, Arnold, 21614 Buxtehude (DE); Bechtel, Martin, 21423 Winsen / Luhe (DE); Eifler, Martin, 25348 Glückstadt (DE); Gardein, Joachim, E-38430 Icod de los Vinos Tenerife/Islas Canarias (ES)
(74) Vertreter: Marx, Thomas

(56) Entgegenhaltungen:
- EP-A1- 2 954 920
- EP-A1- 3 369 451
- EP-A1- 3 375 476
- WO-A1-2016/139623
- DE-A1-102005 041 716
- US-A1- 2004 045 551
- US-A1- 2005 005 940
- US-A1- 2011 048 425

## Beschreibung

Die Erfindung betrifft eine Atemmaske mit einem Maskenkörper und einem gelenkigen Anschlussstück, welches mit einem Atemschlauch verbindbar ist.

Atemmasken werden beispielsweise im Zusammenhang mit Beatmungsgeräten verwendet, um Atemgas zum Patienten zu leiten und eine Ableitung des ausgeatmeten Atemgases zu unterstützen. Die Atemmaske ist typischerweise über einen Atemschlauch mit einem Beatmungsgerät verbunden.

Ein Nachteil bekannter Atemmasken war, die beim Ausatmen durch den Maskenkörper und den Beatmungsschlauch entstehende, für den Patienten und die Umgebung unangenehme, akustische Beeinträchtigung. Zudem verursachte der ausgeatmete Luftstrom einen am Patienten entlang streichenden kühlen Luftzug. Diese Herausforderungen wurden durch eine Adaption der Maske gemäß DE 10 2005 041 716 A1 und EP 1 632 262 B1 mittels geeigneter Anordnung und Struktur von Ausatemspalten zwischen Maskenkörper und Sicherungsring gemeistert.

Fertigungs- und wartungstechnischer Nachteil ist, dass die Luft in den bekannten Atemmasken durch Durchbrüche im Maskenkörper hindurch zu den Ausatemspalten geleitet wird. Zudem schwächen Durchbrüche die mechanische Stabilität des Maskenkörpers.

Des Weiteren bestehen die Masken aus vielen Einzelteilen, die zwar eine erhöhte Anpassbarkeit an den Patienten erlauben, jedoch komplex zu montieren und teuer in der Fertigung sind. Bekannte Masken stellen den Raum zur Aufnahme der Atemöffnungen des. Patienten überwiegend innerhalb des Maskenkörpers zur Verfügung. Dies sorgt für einen erhöhten Materialbedarf im Bereich des Maskenkörpers, da dieser ein größeres Volumen umschließen muss.

Weiterhin stellt bei bekannten Atemmasken die Ankopplung der Bänderung zwecks sicherer Fixierung der Maske am Kopf des Patienten während des Aufsetzens der Maske häufig hohe Anforderungen an die Fingerfertigkeit des Patienten, da die Sicht auf die entsprechenden Verbindungsteile nicht gegeben ist.

Die DE 10 2005 041 716 offenbart eine Atemmaske mit einem Maskenwulst, einem Maskenkörper und zumindest einem Flügel der der Aufnahme eines Bänderungsclips dient, wobei der Bänderungsclip eine Kopfbänderung an der Maske fixiert. Der Flügel weist eine trichterförmige Aussparung auf, die seitlich von einem Steg begrenzt ist. Der Steg dient als Rastpunkt für den Clip.

Ein Nachteil dieser Lösung ist, dass der Steg einen länglichen Rastpunkt ausbildet und der Clip daher an beliebigen Stellen des Stegs verrastet werden kann.

Eine Aufgabe der Erfindung ist es eine definierte Ankopplung und Verrastung der Bänderung bereitzustellen.

Die Aufgabe wird gelöst durch eine Atemmaske mit einem Maskenwulst, der die Maske zum Patienten hin abdichtet, einem Maskenkörper und zumindest einem Flügel der der Aufnahme eines Bänderungsclips dient, wobei der Bänderungsclip eine Kopfbänderung an der Maske fixiert, dadurch gekennzeichnet, dass der Flügel eine trichterförmige Aussparung aufweist und der Clip im Bereich eines Rastpunktes der Aussparung verrastet, wobei dem Rastpunkt zugewandt an dem Maskenkörper oder einer Federplatte ein Rastzapfen angeordnet ist, der bevorzugt eine gerundete Oberflächenkontur aufweist.

Die Erfindung ist dadurch gekennzeichnet, dass die Aussparung zwei unterschiedlich stark trichterförmig geformte Bereiche aufweist.

Die Erfindung ist dadurch gekennzeichnet, dass der Rastpunkt im engsten Bereich der trichterförmigen Aussparung angeordnet ist.

Die Erfindung ist auch dadurch gekennzeichnet, dass der Flügel federnd ausgebildet ist.

Die Erfindung ist dadurch gekennzeichnet, dass der Maskenkörper im Bereich des Flügels eine Federplatte aufweist, die benachbart zu der und/oder innerhalb der Aussparung angeordnet ist.

Die Erfindung ist dadurch gekennzeichnet, dass die Federplatte sich an dem Maskenwulst zumindest teilweise anlehnt.

Die Erfindung ist dadurch gekennzeichnet, dass der Maskenkörper Leitstrukturen aufweist, die den Patienten auf die Aussparung und die Fügerichtung der Clips hinweisen.

Die Erfindung ist dadurch gekennzeichnet, dass die Leitstrukturen an der Basis der Federplatte angeordnet sind.

Die Erfindung ist dadurch gekennzeichnet, dass der Trichter den Clip automatisch zum Rastpunkt führt, in dem der Clip durch die Federplatte fixiert wird.

Die Erfindung ist auch dadurch gekennzeichnet, dass der Clip eine Vertiefung aufweist, die im Zusammenspiel mit dem Rastzapfen für das Einrasten des Clips in der vorgesehenen Position am Flügel in dem Rastpunkt sorgt.

Die Erfindung ist dadurch gekennzeichnet, dass der Clip über die Federplatte gleitet, wenn er zum Rastpunkt führt wird.

Atemmaske nach zumindest einem der vorhergehenden Ansprüche gekennzeichnet durch einen Rastzapfen (20) auf der dem Flügel (12) zugewandten Seite der Federplatte (18), der das Einrasten des Bänderungsclips (6) ermöglicht.

Atemmaske nach zumindest einem der vorhergehenden Ansprüche, die eine Stirnstütze (2) aufweist.

Atemmaske nach zumindest einem der vorhergehenden Ansprüche, die zwei unterschiedliche mechanische Hilfsstrukturen für eine Ankopplung von Bändern aufweist.

Atemmaske nach zumindest einem der vorhergehenden Ansprüche, deren Stirnstütze (2) durch ihren Aufbau mindestens zwei Optionen zur Ankopplung von Bändern aufweist.

Atemmaske nach zumindest einem der vorhergehenden Ansprüche, deren Bandankopplung durch mindestens einen Clip (6) realisiert ist.

Atemmaske nach zumindest einem der vorhergehenden Ansprüche, deren mechanische Hilfsstruktur zur Bandankopplung durch eine trichterförmige Struktur (19) im Maskenkörper (1) gegeben ist.

Atemmaske nach zumindest einem der vorhergehenden Ansprüche dadurch gekennzeichnet, dass der Clip (6) an seiner Ober- und Unterseite Vertiefungen oder Erhebungen aufweist, die mit Noppen oder Rippen (35) besetzt sind und an einem Ende eine Öse (36) aufweist, durch die ein Halteband gezogen und so befestigt werden kann.

Die Erfindung ist dadurch gekennzeichnet, dass der Clip (6) einen Zapfen (37) mit pilzförmigem Hinterschnitt aufweist.

Die Erfindung ist dadurch gekennzeichnet, dass der Clip (6) einen Zapfen (37) mit pilzförmigem Hinterschnitt aufweist sowie eine als Pfanne ausgeführte Vertiefung (38) im Kopf des Zapfens (37) aufweist.

Die Erfindung ist dadurch gekennzeichnet, dass die Vertiefung im Zusammenspiel mit dem Rastzapfen auf der Federplatte des Maskenkörpers für das Einrasten des Bänderungsclips in der vorgesehenen Position am Flügel des Maskenkörpers sorgt.

Die Erfindung ist dadurch gekennzeichnet, dass die Vertiefung (38) /der Rastzapfen (20) in axialer Richtung des Zapfens (37) axial zentriert ausgeführt sind.

Die Erfindung ist dadurch gekennzeichnet, dass die Vertiefung (38) /der Rastzapfen (20) in axialer Richtung des Zapfens (37) axial unzentriert ausgeführt sind, eine Schwenkbewegung des Clips um die Achse des Zapfens (37) führt dann zu einer Lösung der Verbindung zwischen Vertiefung (38) und Rastzapfen (20).

Eine Aufgabe der Erfindung ist es, die Leitstrukturen für die ausgeatmeten Atemgase zu den Ausatemspalten so anzupassen, dass keine Durchbrüche im Maskenkörper erforderlich sind.

Diese Aufgabe wird erfindungsgemäß dadurch gelöst, dass die Leitstrukturen im durch Aussparungen entstehenden Raum zwischen Maskenkörper und einem Stirnstützenverbinder gebildet werden.

Eine weitere Aufgabe der Erfindung ist es, die Anzahl der benötigten Einzelteile zu reduzieren, um eine kostengünstige Fertigung und eine einfache Montage der Atemmaske zu ermöglichen.

Diese Aufgabe wird erfindungsgemäß dadurch gelöst, dass die erfindungsgemäße Stirnstütze mehrere Funktionen vereint. So sind in einem einzigen Teil die Funktionen der Stirnstütze, des Sicherungsringes und des Kugelgelenks zur Aufnahme eines Anschlussstücks kombiniert. Zusätzlich definiert der Stirnstützenverbinder durch seine Kontur gemeinsam mit dem Maskenkörper eine Leitstruktur für die ausgeatmeten Atemgase eines Patienten.

Die Montage der Atemmaske kann zusätzlich dadurch vereinfacht werden, dass der Maskenkörper rotationssymmetrisch ausgeführt wird, wodurch dieser in zwei zueinander um 180° gedrehten Positionen funktionsgleich verbaut werden kann.

Eine weitere Aufgabe der Erfindung ist es Material und Fertigungsaufwand am Maskenkörper einzusparen.

Diese Aufgabe wird dadurch erfüllt, dass der an den Atemöffnungen des Patienten benötigte Raum innerhalb des Maskenwulstes zur Verfügung gestellt wird.
Weiter ist es eine Aufgabe der Erfindung die Bandankopplung an die Atemmaske zur erleichtern.

Diese Aufgabe wird erfindungsgemäß durch eine Anpassung des mechanischen Aufbaus der Bandankopplung in der Form erfüllt, dass eine Führungsstruktur im Maskenkörper integriert wird, die die auf der Bandseite vorhandene Kopplungsstruktur im Ankopplungsvorgang helfend zum Fixierpunkt führt. Dadurch wird die Ankopplung ohne Sicht wesentlich vereinfacht.

Des Weiteren wird diese Aufgabe auch dadurch erfüllt, dass die Stirnstütze verschiedene Optionen für die Bandankopplung bietet.

Die erfindungsgemäße Atemmaske, die nicht nur als Einzelteil, sondern auch als Element eines ganzen Beatmungsgerätes zu verstehen ist, ist bevorzugt modular aufgebaut. Diese Modularität umfasst vier wesentliche Bestandteile, die durch einen Maskenkörper, eine Stirnstütze, einen Maskenwulst und ein gelenkiges Anschlussstück gegeben sind. Das Anschlussstück ist mit einem Atemschlauch verbindbar, der das Atemgas zwischen Atemmaske und Beatmungsgerät transportiert. Weiterhin zeichnet sich die erfindungsgemäße Atemmaske durch die Integration von mindestens einem Ausatemspalt aus, der von bestimmten Bereichen der Oberflächen von Maskenkörper und Stirnstütze zwischen diesen begrenzt wird. Zur Fixierung der Atemmaske am Kopf eines Patienten sind Bänder vorgesehen, für die die erfindungsgemäße Atemmaske Ankopplungsstrukturen zur Verfügung stellt.

Der Maskenkörper dient als Basis der Atemmaske und stellt Verbindungsstrukturen zur Stirnstütze, zum Maskenwulst und zu der Bänderung zur Verfügung. Die Verbindungsstrukturen zu Maskenwulst und Stirnstütze sind bevorzugt mechanisch kodiert, um eine definierte Positionierung der Elemente zueinander sicherzustellen. Als Verbindungsform von Maskenkörper und Stirnstütze ist insbesondere an einen Bajonettverschluss mit Einrastfunktion gedacht. Die Verbindung von Maskenkörper und Maskenwulst kann beispielsweise durch eine Steckverbindung mit Hinterschnitt oder Zweikomponentenverklebung realisiert werden.

Eine vorteilhafte Ausführungsform des Maskenkörpers ist es, diesen rotationssymmetrisch auszulegen. So kann der Maskenkörper funktional identisch in zwei zueinander um 180° gedrehten Positionen in der Atemmaske verbaut werden. Jedoch sind auch, zum Beispiel zwecks mechanischer Kodierung oder der Gestaltung von Ausatemspalten, nicht vollständig rotationssymmetrische Maskenkörper für eine erfindungsgemäße Atemmaske denkbar.

An seinen Seiten weist der Maskenkörper Ankopplungsstrukturen für Bänder auf. Insbesondere ist dabei an die Kombination einer Führungsstruktur, die das Gegenstück zur Ankopplung auf Bandseite zum Fixierpunkt führt, mit einer Fixiermechanik gedacht. Die Fixiermechanik kann erfindungsgemäß beispielsweise als Federplatte mit einem Zapfen ausgeführt sein, während eine trichterförmige Führungsstruktur das bänderseitige Koppelstück in den durch Federplatte und Zapfen definierten Rastpunkt führt.

Die Grundform der inneren Oberfläche des Maskenkörpers ist zum Beispiel ringförmig ausgelegt und öffnet sich auf der einem Patienten abgewandten Seite trichterförmig. Weiterhin weist die Oberfläche im inneren Radius des Maskenkörpers Strukturen auf, die der mechanischen Sicherung der Verbindung mit der Stirnstütze und der Begrenzung von einer oder mehreren Atemgas-Leitstrukturen sowie mindestens einem Ausatemspalt dienen. Diese Strukturen können beispielsweise durch Distanzrippen gegeben sein, die eine Verbindung von Maskenkörper und Stirnstütze ohne Spiel erlauben und die Ausatemspalte begrenzen. Weiterhin sind Strukturen vorgesehen, die eine sichere Verbindung und Fixierung von Maskenkörper und Stirnstütze ermöglichen. Insbesondere ist dabei an kanalartige Aussparungen in zur radialen Ebene orthogonaler Richtung gedacht, die als Führung für Bajonettzähne am Verbindungsstück der Stirnstütze dienen. Des Weiteren dienen die nach dem Verschrauben von Maskenkörper und Stirnstütze freiwerdenden Kanäle in einer vorteilhaften Ausführungsform der erfindungsgemäßen Atemmaske als Leitung für die ausgeatmeten Atemgase eines Patienten vom Inneren der Atemmaske zum Trichter.

Die trichterförmige Öffnung erlaubt es die ausgeatmeten Atemgase eines Patienten in einem vorteilhaften Winkel durch mindestens einen Ausatemspalt an der vom Patienten abgewandten Seite aus der Atemmaske abzuleiten.

Vorteilhaft ist eine Formgebung innerhalb der trichterförmigen Öffnung des Maskenkörpers, die Ausatemspalte in Verbindung mit der montierten Stirnstütze in definierten Bereichen automatisch verschließt. Zum Beispiel ist dabei an, den Bereich gedacht, in dem Ausatemgase direkt oder abgelenkt durch Teile der Atemmaske in Richtung der Augen austreten können, da dies von Patienten als besonders unangenehm wahrgenommen wird.

Jedoch ist auch an Ausführungsformen gedacht, die im nach oben gerichteten Bereich des Trichters mindestens einen Ausatemspalt aufweisen. Auch können Ausführungsformen der erfindungsgemäßen Atemmaske zusätzlich die weitere Struktur der Stirnstütze insbesondere im Bereich des Stegs als führende Oberfläche für das entweichende Atemgas nutzen.

Der Maskenwulst dient der Aufnahme der Atemöffnungen und des Abdichtens der Maske am Gesicht eines Patienten. Der Maskenwulst kann aus einem relativ weichen Material gefertigt sein, das zum Zwecke der Abdichtung am Gesicht eines Patienten und zur Montage an dem Maskenkörper eine bestimmte Elastizität aufweisen kann. Besonders ist an Kunststoff als Material des Maskenwulstes gedacht. Die Grundform des, Maskenwulstes ist bevorzugter Weise an die menschliche Anatomie angepasst und für Nasalmasken entsprechend triangulär vorgesehen. An der vom Patienten abgewandten Seite befindet sich eine Verbindungsöffnung, die in Größe und Form an die Verbindungsform des Maskenkörpers angepasst ist. Durch die Kombination von verschiedenen Formen bei weiterhin angepassten Umfängen auf Seiten von Maskenwulst und Maskenkörper, lassen sich im relativ weichen Material des Maskenwulstes die Steifigkeit und das Verhältnis von Breite und Höhe einstellen. Denkbar sind zum Beispiel eine ovale Ausführung der Wulstaufnahme am Maskenkörper und eine kreisrunde Ausführung der Öffnung am Maskenwulst.

Die Stirnstütze der erfindungsgemäßen Atemmaske ist einteilig ausgeführt und vereint weitere Funktionen. Diese Funktionen sind die Vervollständigung der Leitstruktur für die ausgeatmeten Atemgase eines Patienten in Kombination mit den im Maskenkörper angelegten Merkmalen, insbesondere der trichterförmigen Zuleitung der Atemgase zu dem mindestens einen Ausatemspalt, die gelenkige Anbindung des Anschlussstückes, beispielsweise durch einen integrierten Kugelkäfig, sowie eine Bandankopplung.

Die Erfindung wird im Folgenden anhand der Figuren in beispielhafter Ausführungsform erläutert. Es zeigen:
Fig. 1: eine als Nasalmaske ausgeführte Atemmaske in seitlicher Ansicht,
Fig. 2 : die in Fig. 1 gezeigte Atemmaske von vorn,
Fig. 3 : die in Fig. 1 gezeigte Atemmaske von hinten,
Fig. 4 : die in Fig. 1 gezeigte Atemmaske von oben,
Fig. 5: die in Fig. 1 gezeigte Atemmaske von unten,
Fig. 6: einen vertikalen Schnitt der in Fig. 1 gezeigten Atemmaske,
Fig. 7: eine perspektivische Darstellung einer erfindungsgemäßen Ausführungsform eines Maskenkörpers von vorn,
Fig. 8: eine perspektivische Darstellung des in Fig. 7 gezeigten Maskenkörpers von hinten,
Fig. 9: eine seitliche Ansicht des Maskenkörpers aus Fig. 7,
Fig. 10: eine Ansicht des Maskenkörpers aus Fig. 7 von oben,
Fig. 11: eine Ansicht des Maskenkörpers aus Fig. 7 von vorn,
Fig. 12: eine Ansicht des Maskenkörpers aus Fig. 7 von hinten,
Fig. 13: einen vertikalen Schnitt durch den Maskenkörper aus Fig. 7,
Fig. 14: einen horizontalen Schnitt durch den Maskenkörper aus Fig. 7,
Fig. 15: eine perspektivische Ansicht einer erfindungsgemäßen Ausführungsform der Stirnstütze von vorn,
Fig. 16: eine perspektivische Ansicht einer Stirnstütze aus Fig. 15 von hinten,
Fig. 17: eine Ansicht der Stirnstütze aus Fig. 15 von unten,
Fig. 18: eine Ansicht der Stirnstütze aus Fig. 15 von oben,
Fig. 19: eine perspektivische Ansicht eines Moduls einer erfindungsgemäßen Beatmungsvorrichtung bestehend aus Maskenkörper und Stirnstütze,
Fig. 20: eine Seitenansicht des in Fig. 19 gezeigten Moduls,
Fig. 21: eine perspektivische Ansicht eines erfindungsgemäßen Anschlussstücks,
Fig. 22: eine seitliche Ansicht eines erfindungsgemäßen Anschlussstücks,
Fig. 23: einen vertikalen Schnitt des Anschlussstücks aus Fig. 21,
Fig. 24: eine perspektivische Darstellung eines erfindungsgemäßen Clips für die Bandaufnahme und die Verbindung mit dem Maskenkörper von der der Maske abgewandten Seite,
Fig. 25: eine perspektivische Darstellung des Clips aus Fig. 24 von der der Maske zugewandten Seite.

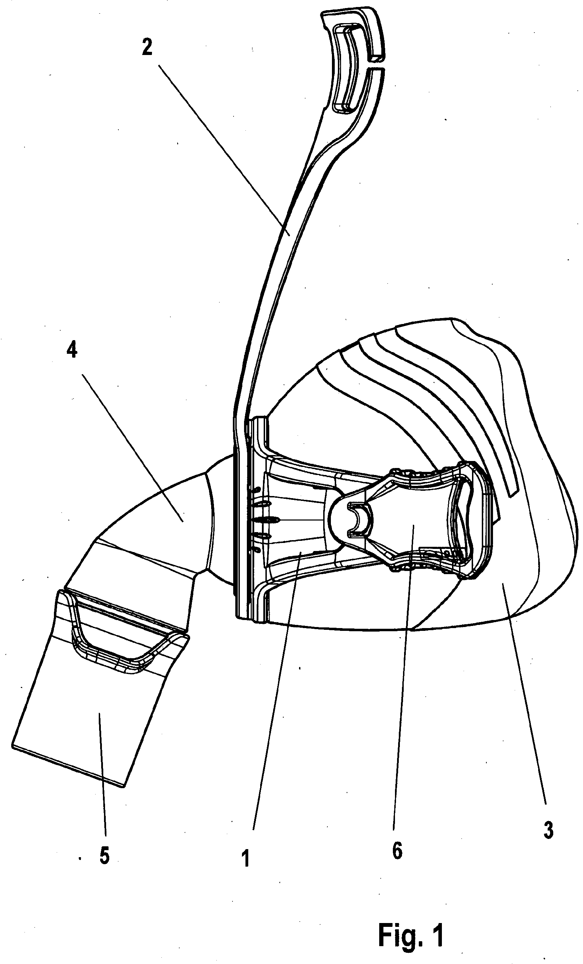

Fig. 1 zeigt eine als Nasalmaske ausgebildete Atemmaske. Die erfindungsgemäße Atemmaske ist modular aufgebaut. Als Basis der Atemmaske dient der Maskenkörper (1). Dieser ist beispielsweise als Spritzgussteil aus Kunststoff relativ fest ausgeführt. An dem Maskenkörper (1) setzen die Stirnstütze (2), die einen sicheren Sitz der Maske auf dem Gesicht eines nicht dargestellten Patienten gewährleistet, und der Maskenwulst (3), der durch seine Passform und seine elastische Beschaffenheit für einen dichten Abschluss der Atemmaske am Patienten sorgt, an. Das Anschlussstück (4) stellt die Verbindung zu einem nicht dargestellten Schlauch dar, über den das Atemgas von einem Beatmungsapparat zur Atemmaske transportiert wird. Der Schlauch wird mittels einer auf dem Anschlussstück (4) drehbeweglich gelagerten Hülse (5) mit dem Anschlussstück (4) verbunden. Mithilfe von seitlich mit dem Maskenkörper (1) verbindbaren Clips (6) kann die Atemmaske durch Bänder sicher am Kopf des Patienten fixiert werden.

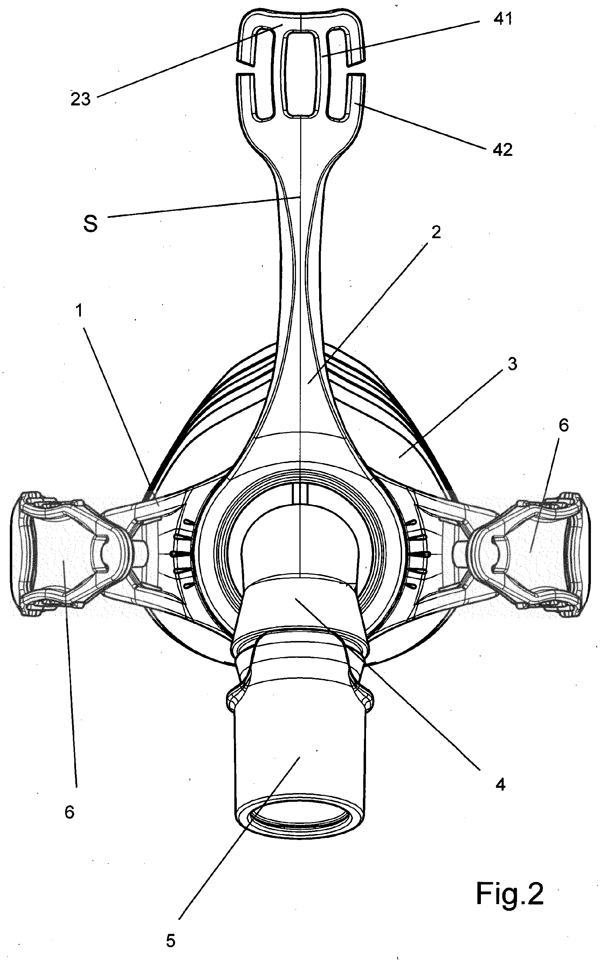

Fig. 2 zeigt die bereits in Fig. 1 dargestellte Ausführungsform einer erfindungsgemäßen Atemmaske von vorn. Zu erkennen ist die Symmetrie des Aufbaus entlang der vertikalen Mittelachse. An beiden Seiten des Maskenkörpers (1) befindet sich hier ein Clip (6) zur Bandaufnahme, jedoch ist auch eine asymmetrische Ausführungsform denkbar. Dies kann beispielsweise umgesetzt werden, indem auf einer Seite die Verbindung von Atemmaske und Halteband mit einem Clip (6) realisiert wird, während auf der anderen Seite eine Schlaufe und ein Haken genutzt werden.

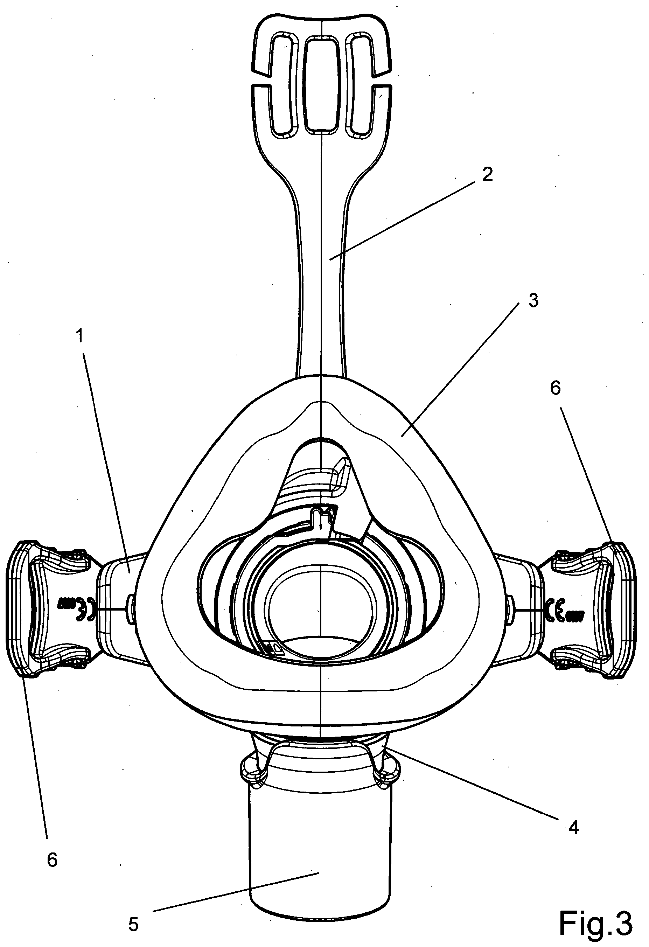

Fig. 3 zeigt die Atemmaske aus Fig. 1 von hinten. Zu sehen ist eine trianguläre Grundform des Maskenwulstes (3) sowie das Innere der Atemmaske durch die patientenseitige Öffnung des Maskenwulstes (3) hindurch. An der oberen Seite des Maskenwulstes (3) ragt die Stirnstütze (2) hervor. An den Seiten der Atemmaske sind die mit dem Maskenkörper (1) verbundenen Clips (6) zur Bandankopplung zu sehen. An der unteren Seite des Maskenwulstes (3) ragt das Anschlussstück (4) mit der montierten Hülse (5) hervor. Die Clips überragen den Maskenkörper in Richtung auf den Patienten. Damit verlängern die Clips den Maskenkörper (1) in Richtung auf den Patienten. Daraus ergibt sich, dass die Angriffpunkte für die Bänderung näher zu den Ohren des Patienten verschoben werden.

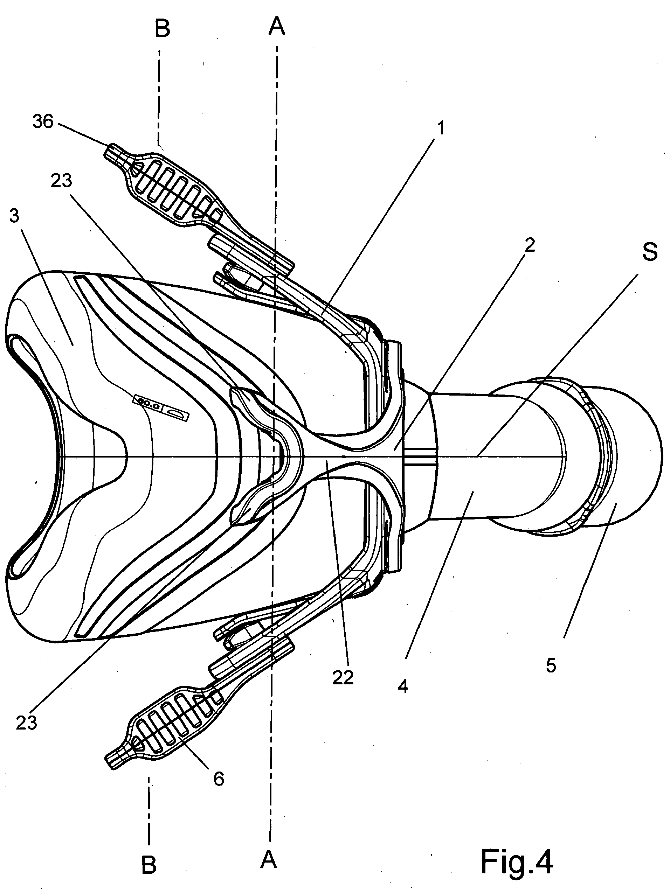

Fig. 4 zeigt die Atemmaske aus Fig. 1 von oben. In dieser Ansicht ist eine der menschlichen Anatomie angepasste Formgebung der Stirnstütze (2) erkennbar. Aus der sich aus der Verbindung mit dem Maskenkörper (1) ergebenden Fläche ragt das Kopfende der Stirnstütze (2) in Richtung eines Patienten, um auch ohne übermäßiges Neigen der gesamten Atemmaske eine ausreichende Stützfunktion an der Stirn eines Patienten zu erreichen. Eingezeichnet ist die Lage der Öse (36) die der Ankopplung der Bänderung an den Clip dient. Zu erkennen ist auch die Bandankopplung im Bereich der Stirnstütze (23). Durch den symmetrischen Aufbau relativ zu der Symmetrieebene S sind die Ösen (36) und die Bandankopplung (23) beidseitig vorhanden. Eine Ebene A verläuft durch beide Bandankopplungen (23) (und im Wesentlichen parallel zur Stirn eines Patienten) . Eine Ebene B verläuft durch beide Ösen (36) (und im Wesentlichen parallel zur Stirn eines Patienten). Die Ebene A ist weiter von dem Patienten (Stirn eines Patienten) als Ebene B. Die Anknüpfpunkte der Bänderung liegen in den Ebenen A bzw. B. Die Anknüpfpunkte der Bänderung sind daher bei den Clips in einer anderen Ebene als die Anknüpfpunkte der Bandankopplung (23) der Stirnstütze.

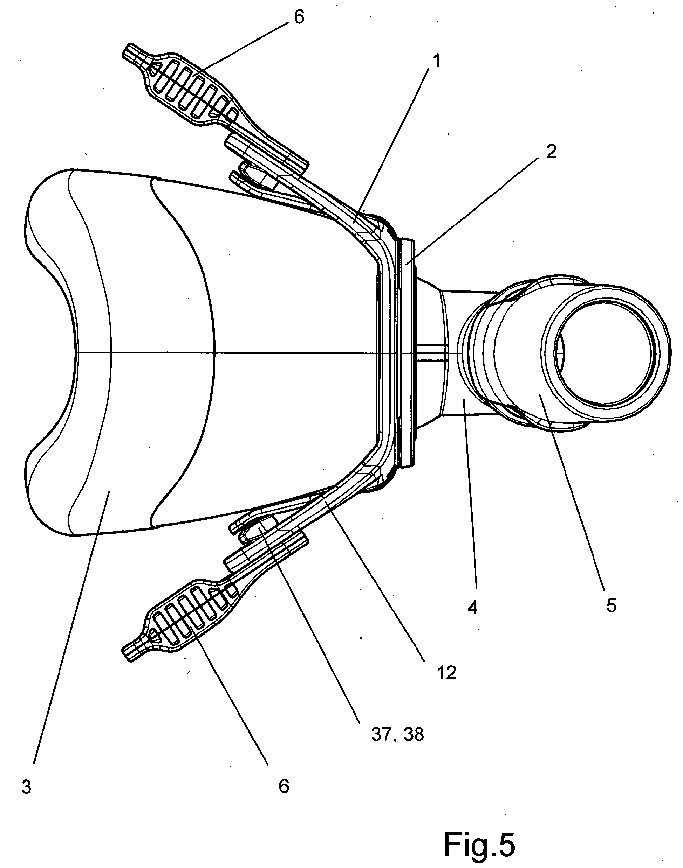

In Fig. 5 ist die in Fig. 1 gezeigte Atemmaske von unten dargestellt. Zu sehen sind der Maskenkörper (1), die Stirnstütze (2), der Maskenwulst (3) auf der einem Patienten zugewandten Seite, das Anschlussstück (4) auf der einem Patienten abgewandten Seite mit montierter Hülse (5) und, Clips (6) zur Bandankopplung an die Atemmaske.

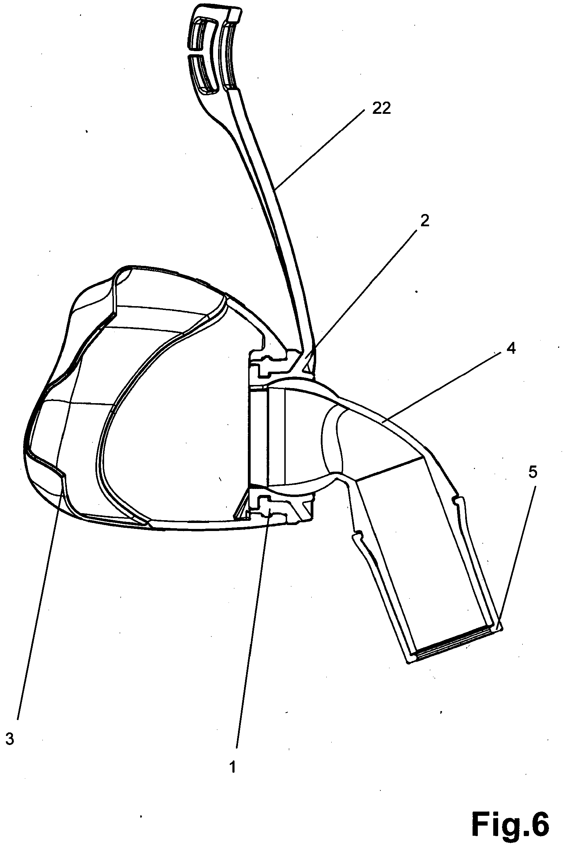

Fig. 6 zeigt einen vertikalen Schnitt durch die Mitte der in Fig. 1 gezeigten Atemmaske. Zu sehen sind der Maskenkörper (1), die Stirnstütze (2), der Maskenwulst (3) auf der einem Patienten zugewandten Seite und das Anschlussstück (4) mit montierter Hülse (5) auf der einem Patienten abgewandten Seite der Atemmaske.

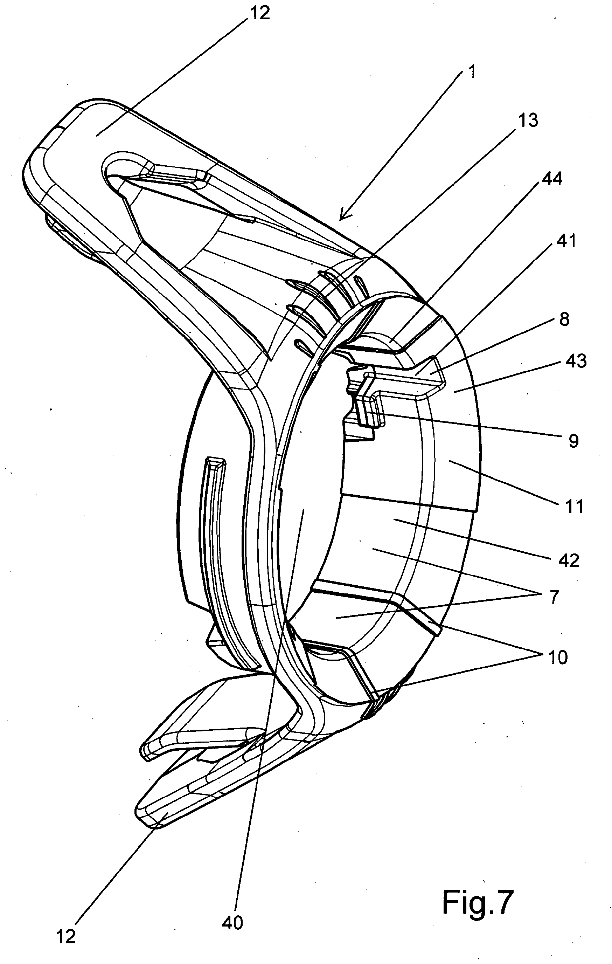

In Fig. 7 wird eine perspektivische Darstellung des Maskenkörpers (1) gezeigt. Zu erkennen sind mehrere Ausströmflächen (7) auf der innenliegenden Oberfläche des Maskenkörpers (1). Die innenliegende Oberfläche des Maskenkörpers (1) öffnet sich nach vorn trichterförmig, während ihre Grundform im hinteren, einem Patienten zugewandten Bereich durch ein Rohr konstanten Durchmessers definiert wird. In der inneren Kontur des Maskenkörpers (1) befinden sich mindestens zwei Kanäle (8), die als Aussparung im rohrförmigen Bereich realisiert sind und die sich bis in den trichterförmigen Bereich erstrecken. Diese Kanäle (8) fungieren in doppelter Funktion sowohl als Aufnahmen für die mechanische Ankopplung der Stirnstütze (2), die beispielsweise als Bajonettverschluss realisiert werden kann, als auch als Abströmkanäle für ausgeatmete Atemgase, deren vollständige Kontur gemeinsam von Maskenkörper (1) und Stirnstütze (2) definiert wird. Für den Bajonettverschluss ist im hinteren Bereich neben jedem Kanal (8) jeweils ein Zahn (9) vorgesehen, der radial nach innen aus der inneren Kontur des Maskenkörpers (1) herausragt und der den benötigten mechanischen Widerstand für den Verschluss bietet. Des Weiteren sind auf der inneren Oberfläche des Maskenkörpers (1) mehrere Distanzrippen (10) angeordnet, die eine Verbindung von Maskenkörper (1) und Stirnstütze (2) ohne Spiel bzw. unter Vorspannung ermöglichen. Im Bereich (11) der Abströmkanäle (8) ist das Material im Ring- und Trichterbereich entsprechend der Höhe der Distanzrippen (10) verstärkt. Die Distanzrippen (10) und die verstärkten Bereiche (11) begrenzen zudem seitlich die Ausströmflächen (7). Denkbar sind auch zusätzliche Aussparungen im trichterförmigen Bereich der inneren Oberfläche des Maskenkörpers (1), um einen oder mehrere zusätzliche Ausströmkanäle zu formen.

An seinen Seiten weist der Maskenkörper (1) zwei Flügel (12) auf, die jeweils eine mechanische Struktur zur Aufnahme einer Bandankopplung aufweisen. Die Bandankopplung kann beispielsweise durch einen Clip (6) erfolgen. Im vorderen Bereich des Maskenkörpers (1) ist dieser auf Höhe der Flügel (12) mit Noppen (13) versehen, die eine bessere Griffigkeit bei der Handhabung der Atemmaske ermöglichen.

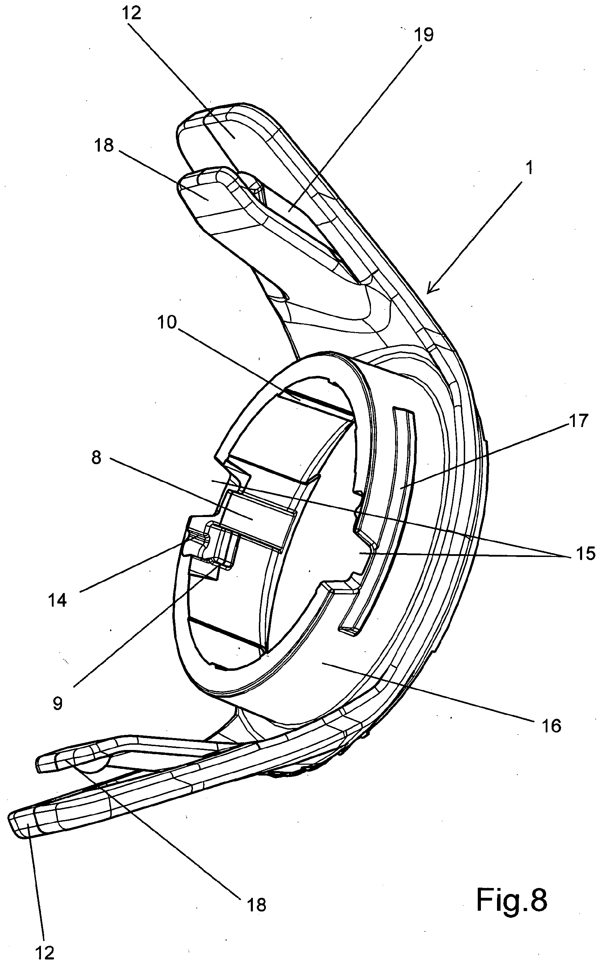

Fig. 8 zeigt eine perspektivische Darstellung des Maskenkörpers (1) von hinten. In dieser Darstellung ist eine Ausführung der im Maskenkörper (1) angelegten Struktur für den Bajonettverschluss zu erkennen. Der Kanal (8) und wird in seiner Grundform hinter dem Zahn (9) zu einer L-förmigen Aussparung ergänzt, was die typische Steck- und Schraubverbindung ermöglicht. Im Konturbereich hinter dem Zahn (9) ist ein Rastzahn (14) vorgesehen, der die Verbindung sichert. Zusätzlich ist in der in Fig. 8 gezeigten Ausführungsform der Atemmaske eine mechanische Kodierung (15) im Maskenkörper (1) derart realisiert, dass der Maskenwulst (3) sich nur in vorgesehenen Positionen mit dem Maskenkörper (1) verbinden lässt. Diese Verbindung erfolgt durch ein Aufstecken des Maskenwulstes (3) auf die äußere Kontur (16) des Maskenkörpers (1) . Gesichert wird die Verbindung in der gezeigten Ausführungsform durch mindestens einen Hinterschnitt (17) auf der äußeren Kontur (16) des Maskenkörpers (1) . Denkbar sind neben der dargestellten kreisrunden äußeren Kontur (16) auch andersförmige Ausführungen. Insbesondere ist an eine ovale Form der äußeren Kontur (16) gedacht. In Kombination mit der Form der Verbindungsöffnung des Maskenwulstes (3), die mit einem inneren Umfang kleiner oder gleich der äußeren Kontur (16) des Maskenkörpers (1), auch anders geformt sein kann, können die Steifigkeit und das Verhältnis von Höhe und Breite des Maskenwulstes (3) eingestellt werden. Insbesondere ist an eine Kombination von ovaler Ausführung der äußeren Kontur (16) und einer kreisrunden Ausführung der Verbindungsöffnung des Maskenwulstes (3) gedacht. Neben der gezeigten Verbindung von Maskenwulst (3) und Maskenkörper (1), durch einen Hinterschnitt (17), sind auch andere Optionen denkbar. Zum Beispiel ist auch ein Verkleben der Module mit Hilfe eines Zweikomponentenklebstoffs möglich.

Die von im Bereich der Flügel (12) bereitgestellte mechanische Aufnahmestruktur für die Bandankopplung besteht im Wesentlichen aus einer Federplatte (18) in Kombination mit einer Aussparung (19) in der Struktur des Flügels (12). Die Aussparung (19) ist trichterförmig. Dabei kann die Aussparung (19) einen ersten Abschnitt mit mäßiger Trichterform (19a) und einen zweiten Abschnitt mit deutlicher Trichterform (19b) aufweisen. Die Aussparung (19) mündet in einem Rastbereich (39) der der Form des Clips (6, 37,38) zumindest abschnittsweise angepasst ist. Der Maskenkörper weist zusätzlich Leitstrukturen (13) auf, die den Patienten auf die Aussparung (19) bzw. die Fügerichtung der Clips hinweisen. Alternativ können die Leitstrukturen (13) auch als mechanische Strukturen (Rinnen, Kanäle, Kanten oder dergleichen) ausgebildet sein, die den Clip in Richtung der Aussparung leiten.

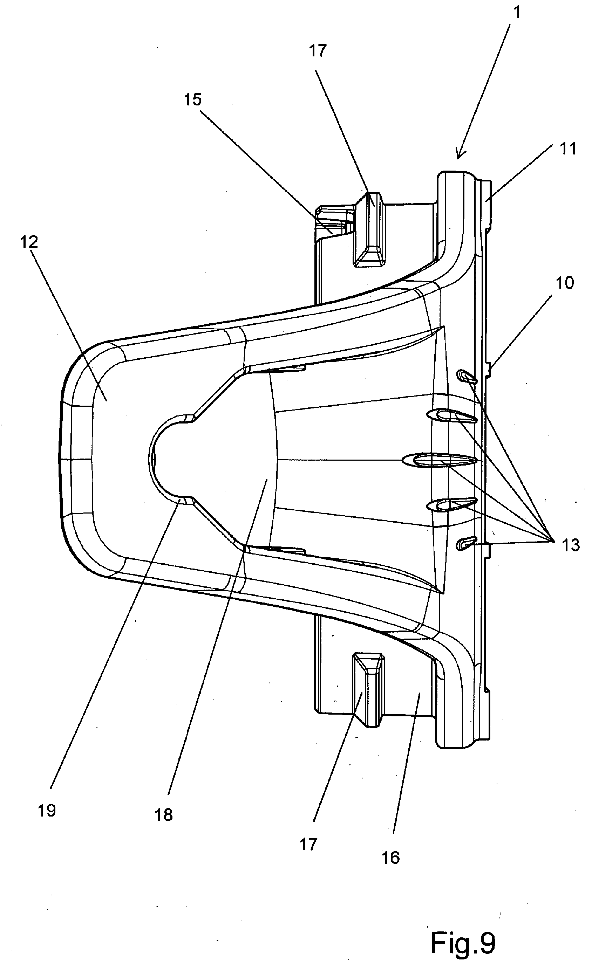

Fig. 9 zeigt eine Seitenansicht des Maskenkörpers (1) . Zu sehen ist eine trichterförmige Umsetzung der Aussparung (19) im Flügel (12), die ein benutzerfreundliches Einführen der Bandankopplung, zum Beispiel in Form eines Bänderungsclips (6), ermöglicht. Dies ist erforderlich, da sich dieser Bereich bei Nutzung oder Anlegen der Atemmaske nicht im Sichtfeld des Patienten befindet. Der Trichter (19) führt den Clip (6) automatisch zum Rastpunkt, in dem der Clip (6) durch die Federplatte (18) fixiert wird.

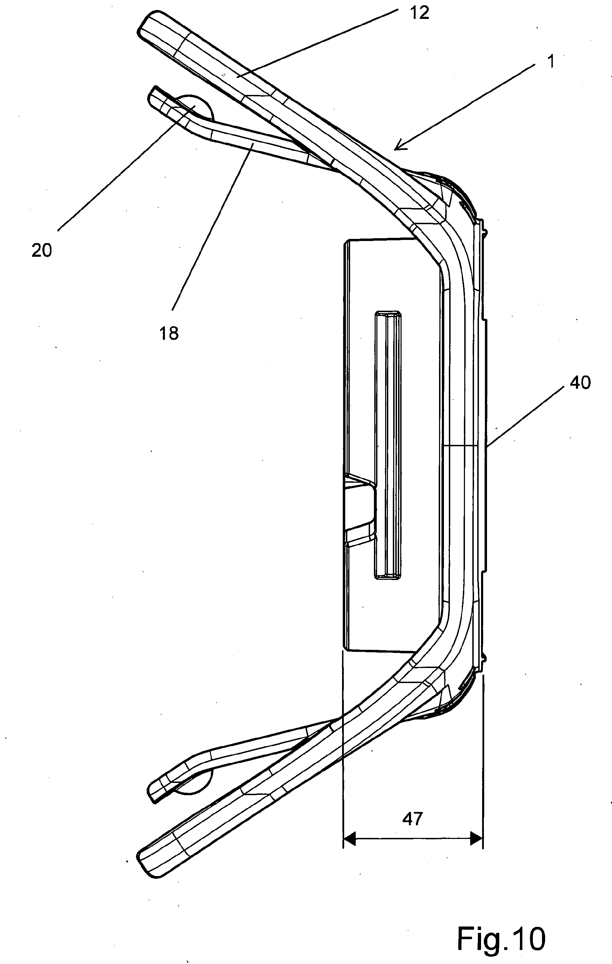

Fig. 10 zeigt eine erfindungsgemäße Ausführungsform des Maskenkörpers (1) von unten. Zu erkennen ist ein Rastzapfen (20) auf der dem Flügel (12) zugewandten Seite der Federplatte (18), der das Einrasten des Bänderungsclips (6) ermöglicht.

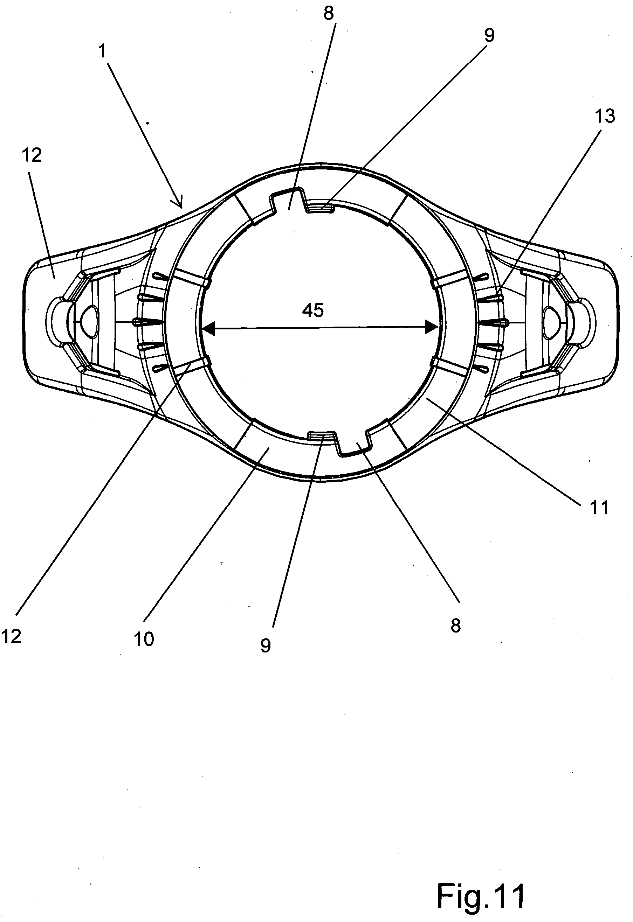

Fig. 11 zeigt den in Fig. 7 dargestellten Maskenkörper (1) in frontaler Ansicht. Zu erkennen ist die Rotationssymmetrie um eine im Mittelpunkt des inneren Radius befindlichen, senkrecht aus der Zeichenebene herausragenden Achse um 180°.

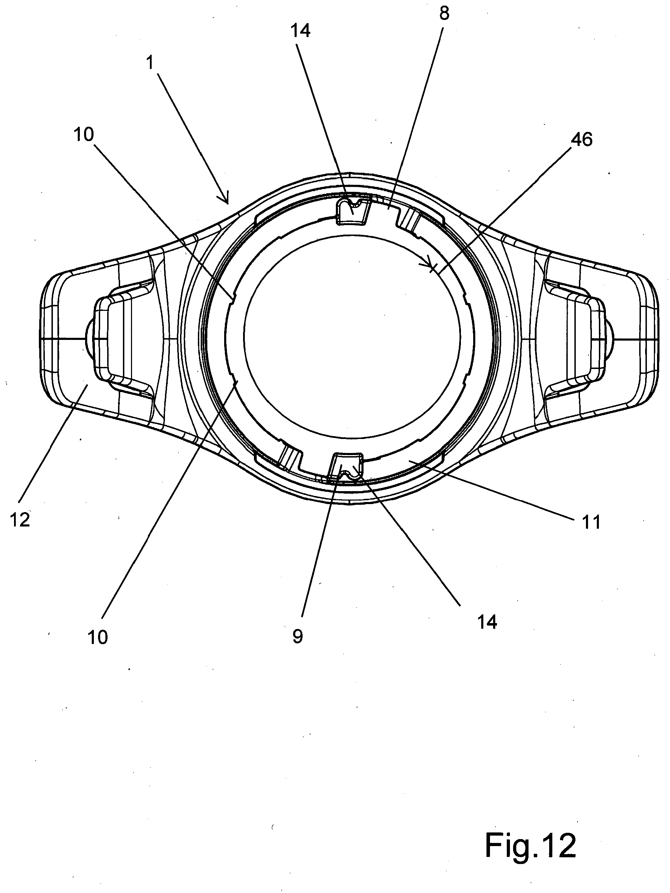

Fig. 12 zeigt den in Fig. 7 dargestellten Maskenkörper (1) von hinten. Ersichtlich sind die radial aus der inneren Kontur (7) herausragenden Distanzrippen (10) und die verstärken Bereiche (11) um die Ausströmkanäle (8). Auch ist die Form der Rastzahns (14) für den Bajonettverschluss zu sehen, der die Bajonettverbindung durch die Verrastung mit dem Gegenstück an der Stirnstütze (2) gegen ungewolltes Verdrehen und Lösen sichert.

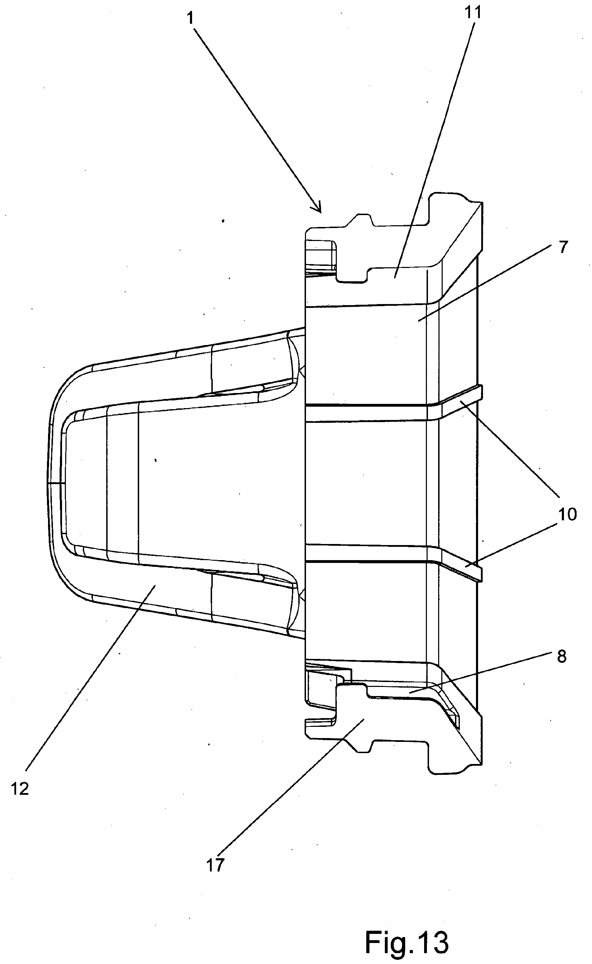

Fig. 13 zeigt einen vertikalen Schnitt durch die Mitte des in Fig. 7 gezeigten Maskenkörpers (1). Zu sehen ist die trichterförmige Öffnung der inneren Kontur des Maskenkörpers (1) zur rechten Seite. Im zwischen den verstärkten Bereichen (11) um die Ausströmkanäle (8) liegenden Bereich der inneren Oberfläche des Maskenkörpers (1), sind jeweils zwei Distanzrippen (10) in gleichen Abständen zueinander und zu den verstärkten Bereichen (11) angeordnet. Die Anzahl und Positionierung der Distanzrippen (10) kann in erfindungsgemäßen Ausführungsformen der Atemmaske variieren.

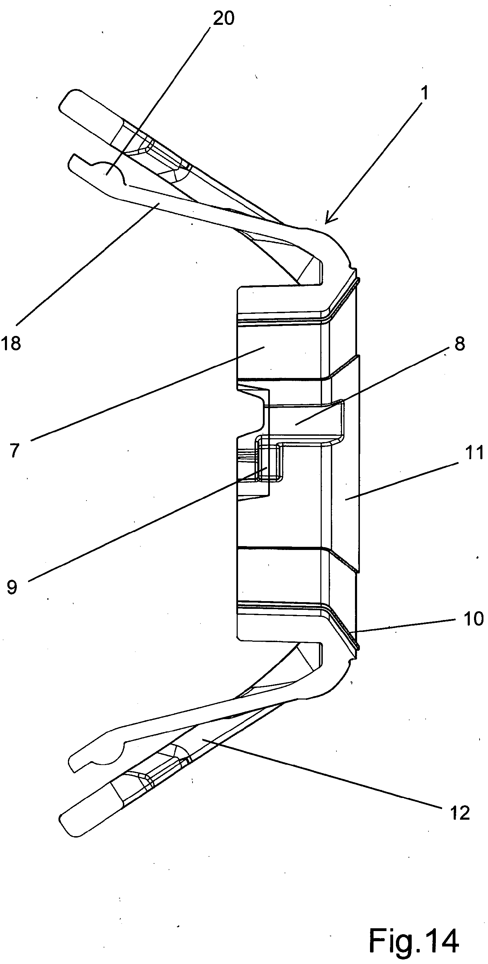

Fig. 14 zeigt einen horizontalen Schnitt durch die Mitte des in Fig. 7 dargestellten Maskenkörpers (1) . Zu sehen ist die Positionierung von Federplatte (18) mit Rastzapfen (20) zum Grundkörper des Flügels (12). Der Abstand von Federplatte (18) und Grundkörper des Flügels (12) ist auf einen Clip (6) angepasst.

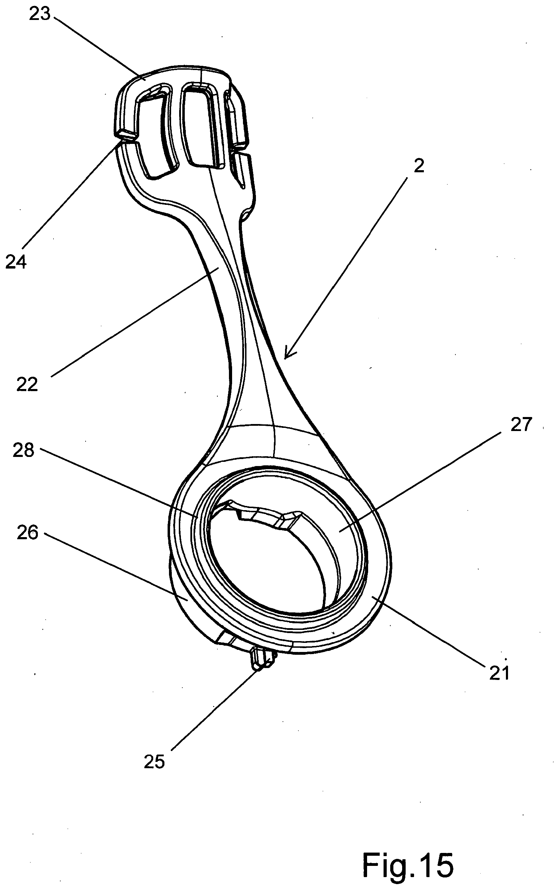

Fig. 15 zeigt eine erfindungsgemäße Ausführungsform einer Stirnstütze (2). Die Basis der Stirnstütze (2) bildet der Stirnstützenverbinder (21), mit dem die Stirnstütze (2) am Maskenkörper (1) befestigt wird. Ein Steg (22) verbindet den Stirnstützenverbinder (21) mit der Bandankopplung (23) am Kopf der Stirnstütze (2) . Die Bandankopplung (23) besteht aus drei horizontal nebeneinander angeordneten, in vertikaler Richtung länglich ausgedehnten Aussparungen in der Struktur der Stirnstütze (2). Die beiden äußeren Aussparungen weisen jeweils einen zusätzlichen Spalt (24) in der nach außen gewandten Struktur der Stirnstütze (2) auf. Diese Spalte (24) können zum Einfädeln der Haltebänder auf Stirnhöhe eines Patienten in die Bandankopplung (23) genutzt werden. Alternativ kann das Band auch durch die mittig positionierte Aussparung geführt werden.

Es ist auch an andere Ausführungsformen der Bandankopplung (23) gedacht. Beispielsweise kann diese auch nur durch zwei Aussparungen mit zusätzlichem Spalt (24) umgesetzt werden. Der Steg (22) ist in seiner Breite in der gezeigten Ausführungsform gegenüber dem Kopfteil mit Bandankopplung (23) und dem Stirnstützenverbinder (21) signifikant reduziert. Dies ermöglicht ein ansprechendes, schlankes Design und die Einsparung von Material.

Der Stirnstützenverbinder (21) bietet zur Vervollständigung des im Maskenkörper (1) angelegten Bajonettverschlusses eine in Positionierung und Anzahl der im Maskenkörper vorhandenen Kanäle (8) entsprechende Anzahl an Verschlusszähnen (25). Diese stehen auf der äußeren Kontur des Verbindungsringes (26) radial nach außen und weisen ihrerseits jeweils einen weiteren radial nach außen gerichteten Zahn auf, der gemeinsam mit dem Rastzahn (14) im Maskenkörper (1) für die Arretierung von Maskenkörper (1) und Stirnstütze (2) in der vorgesehenen Position sorgt. Die innere Kontur des Stirnstützenverbinders (21) ist als Kugelkäfig (27) ausgeführt, der das Anschlussstück (4) beweglich lagern kann. Zum Anschlussstück (4) hin wird der Kugelkäfig (27) durch eine sich nach außen verengende Ringstruktur (28) begrenzt.

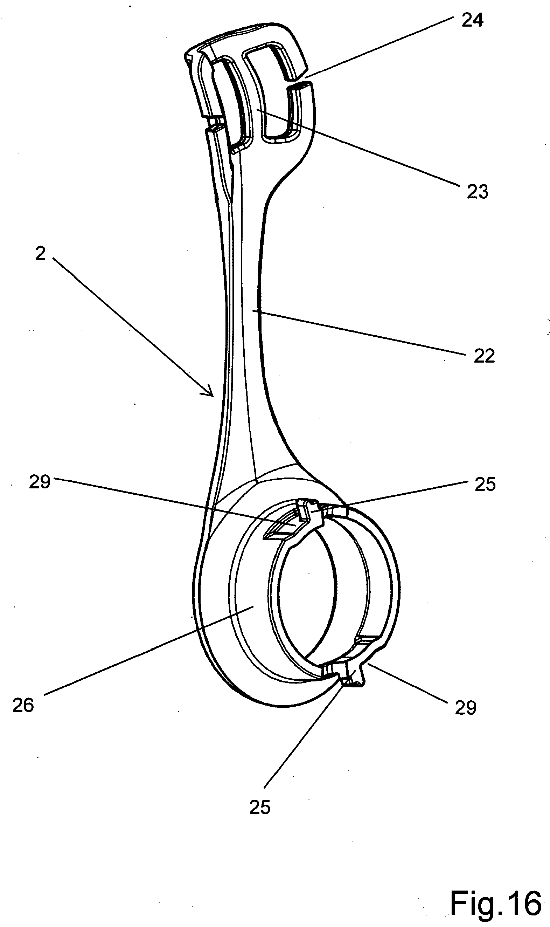

Fig. 16 zeigt eine perspektivische Darstellung einer Stirnstütze (2) von hinten. Die dargestellte Stirnstütze (2) weist zwei Verschlusszähne (25) auf. Jedoch sind auch mehr Verschlusszähne (25) passend zum jeweiligen Maskenkörper (1) denkbar. Erkennbar sind Aussparungen (29) in der äußeren Kontur des Verbindungsringes (26) neben den Verschlusszähnen (25). Diese Aussparungen (29) dienen als zusätzliche Ausströmkanäle und leiten die ausgeatmeten Gase um den jeweiligen Verschlusszahn (25) zum entsprechenden im Maskenkörper (1) angeordneten Kanal (8). Zusätzlich ist auch die Nutzung einer solchen Aussparung (29) als mechanische Kodierung für eine definierte Ausrichtung von Maskenkörper (1), Stirnstütze (2) und Maskenwulst (3) denkbar. Dafür muss die Struktur des Maskenkörpers (1) im Bereich seiner mechanischen Kodierung (15) entsprechend durchlässig sein.

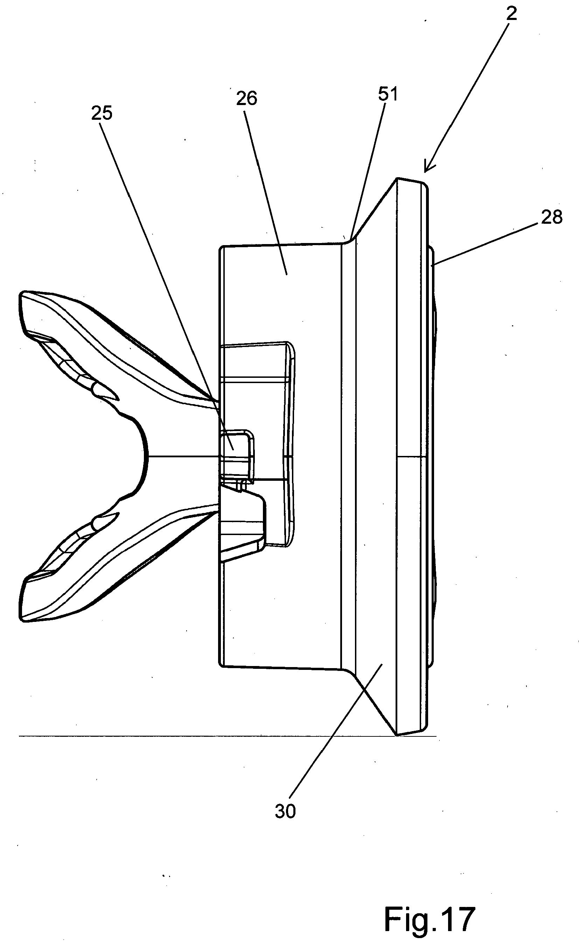

In Fig. 17 ist die Stirnstütze (2) aus Fig. 15 von unten dargestellt. Zu erkennen ist die der inneren Kontur des Maskenkörpers (1) angepasste äußere Kontur des Stirnstützenverbinders (21), die sich ebenfalls aus einem Ring und einem Trichter zusammensetzt. Diese Kontur bildet die stirnstützenseitige Abströmoberfläche (30) und definiert gemeinsam mit der innenliegenden Oberfläche des Maskenkörpers (1) die Form der Abströmkanäle und der Ausatemspalte.

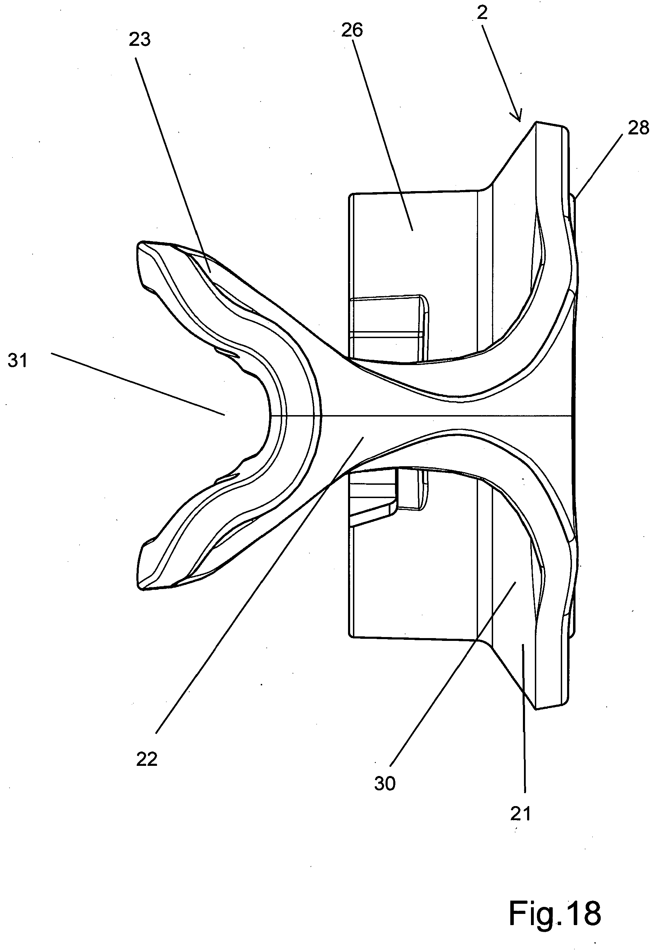

Fig. 18 zeigt die Stirnstütze (2) aus Fig. 15 von oben. Zu erkennen ist, dass das Kopfteil der Stirnstütze (2) in Relation zum Stirnstützenverbinder (21) zu einem Patienten hin ragt. Die Form des die Bandankopplung (23) beherbergenden Kopfes der Stirnstütze (2) weist eine Aussparung (31) auf, die zwischen der Stirn eines Patienten und der Bandankopplung (23) in der Stirnstütze (2) einen Raum bietet, in dem die Bänder liegen können.
Die Form des die Bandankopplung (23) beherbergenden Kopfes der Stirnstütze (2) weist eine Aussparung (31) auf, die zwischen der Stirn eines Patienten und der Bandankopplung (23) in der Stirnstütze (2) einen Raum bietet. Die Aussparung (31) weist gemeinsam mit den Strukturelementen der Bänderungsankopplung, in einer Ansicht von oben eine Y-Form auf. Der Konturverlauf im Bereich der Aussparung (31) verläuft gerundet mit einem oder mehreren Radien. Entsprechend des Materials der Bänderungsankopplung und der Wandstärken kann die Y-Struktur eine Elastizität bieten, die eine Adaptation des Kopfteils der Stirnstütze (13) relativ zur Stirn des Patienten erlaubt.

Die Bandankopplung (23) weist eine Symmetrieebene S auf und zumindest vier Aufnahmemittel (41,42) für ein Bänderungsende einer Bänderung wobei die Aufnahmemittel in Paaren beidseitig der Symmetrieebene S angeordnet sind und ein Bänderungsende alternativ an dem ersten Aufnahmemittel (41) oder an dem zweiten Aufnahmemittel (42) auf einer Seite des Kopplungselementes befestigbar ist. Das Aufnahmemittel 41 ist räumlich benachbart zum Aufnahmemittel 42 und näher zu der Symmetrieebene S angeordnet, als das Aufnahmemittel 42.

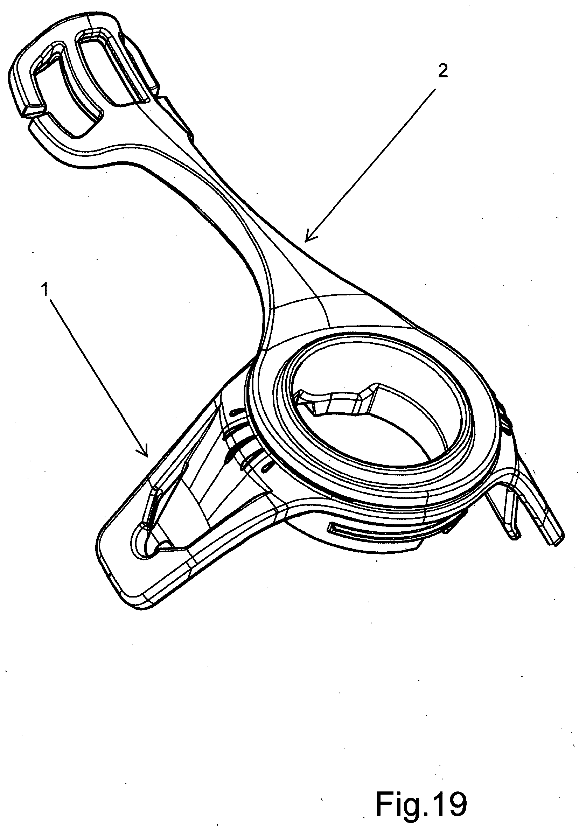

In Fig. 19 sind Maskenkörper (1) und Stirnstütze (2) als montiertes Modul in perspektivischer Darstellung zu sehen. Bei einer rotationssymmetrischen Ausführung des Maskenkörpers (1) kann dieser in genau zwei um 180° verschobenen Positionen mit der Stirnstütze (2) verbunden werden, die funktional identisch sind. Damit lässt sich eine Vereinfachung der Montage der Module der Atemmaske erreichen.

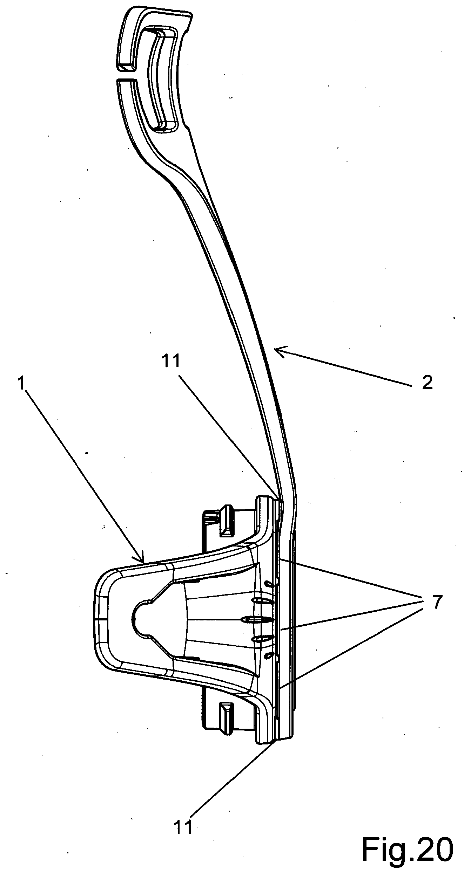

Fig. 20 zeigt eine seitliche Ansicht des montierten Moduls aus Maskenkörper (1) und Stirnstütze (2). Die zwischen den Abströmoberflächen (7) des Maskenkörpers (1), sowie den Ausströmkanälen (8) und der Abströmoberfläche (30) des Stirnstützenverbinders (21) verbleibenden Spalte, dienen als Leitstruktur für die ausgeatmete Luft. Durch die erhöhte Struktur im Bereich (11) der Kanäle (8) im Maskenkörper (1) wird an der oberen Seite in Richtung der Stirnstütze (2) eine Abdichtung des Ausatemspaltes in einem definierten Bereich erreicht. Dies dient der Vermeidung der Ausströmung von Luft in für einen Patienten unangenehme Bereiche, insbesondere die Augen.

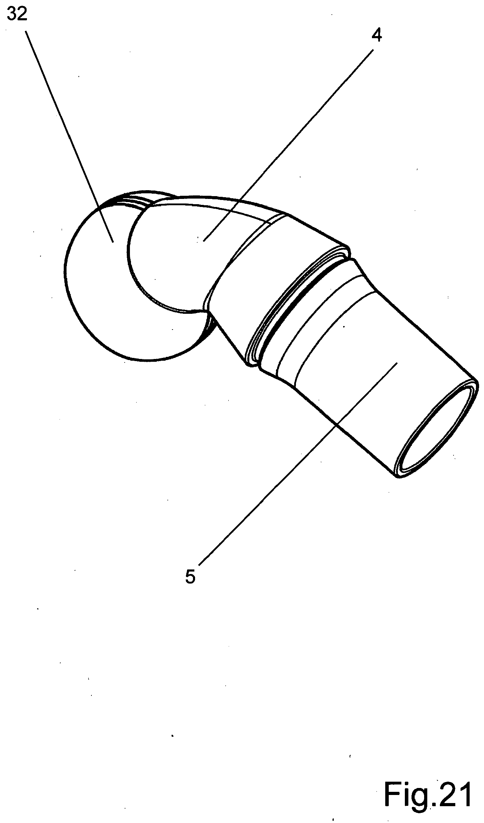

Fig. 21 zeigt eine perspektivische Darstellung eines erfindungsgemäßen Anschlussstückes (4). Das Anschlussstück (4) ist als abgewinkelte Rohrstruktur ausgeführt und weist an einem Ende eine Oberflächenkontur (32) auf, die einer Teilkugel entspricht. Diese Teilkugel (32) dient der beweglichen Verbindung von Anschlussstück (4) und Stirnstütze (2) mittels des im Stirnstützenverbinder (21) realisierten Kugelkäfigs (27).

Am anderen Ende ist eine drehbar gelagerte Hülse (5) als Anschluss für einen nicht dargestellten Schlauch montiert.

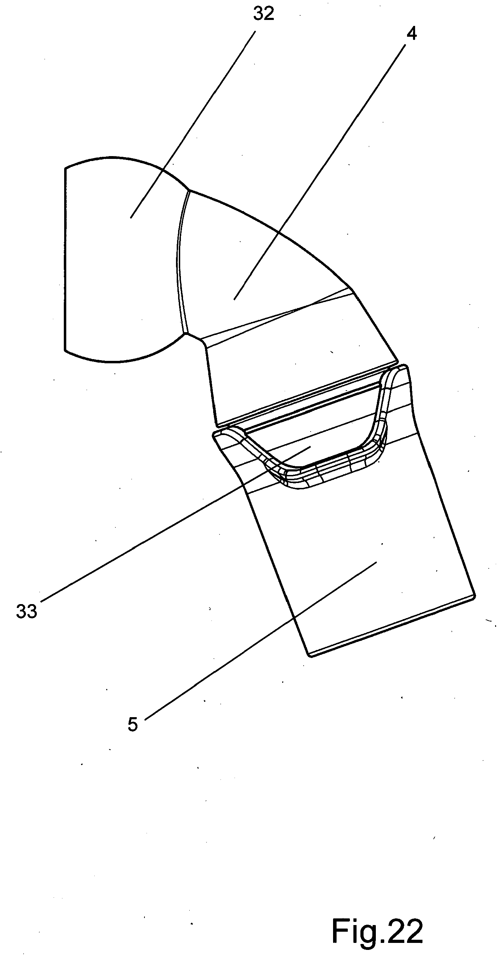

Fig. 22 zeigt eine Seitenansicht eines erfindungsgemäßen Anschlussstückes (4) mit einer alternativen drehbar gelagerten Hülse (5). Die Hülse (5) verdickt sich am zur Atemmaske orientierten Ende und weist an diesem Ende Aussparungen (33) auf.

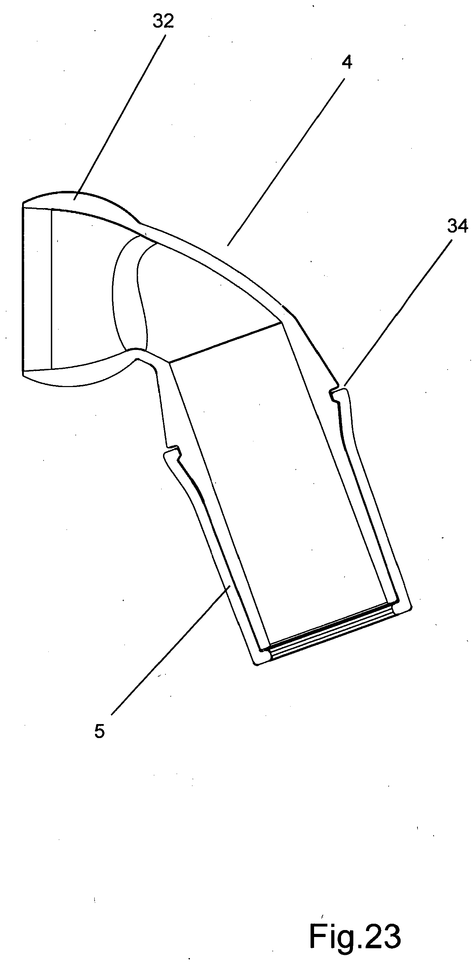

Fig. 23 zeigt einen vertikalen Schnitt durch das Anschlussstück (4). Zu sehen sind am oberen Ende die Oberflächenkontur in Form einer Teilkugel (32), die rohrförmige innere Struktur und eine Verdickung der Struktur hin zu einer ringförmig umlaufenden Nut (34), die die Hülse mittels einem an dieser vorhandenen Hinterschnitt drehbar lagert.

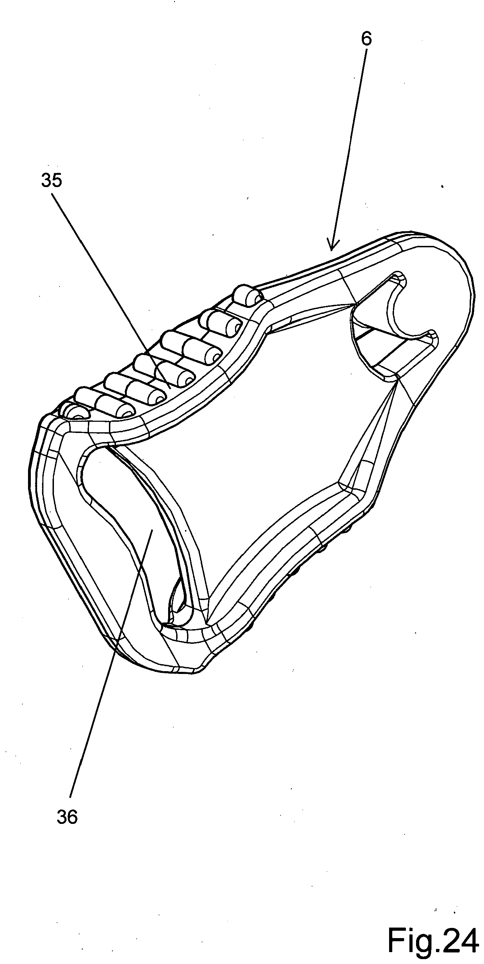

Fig. 24 zeigt eine perspektivische Darstellung eines Bänderungsclips (6). An seiner Ober- und Unterseite weist der Clip (6) Vertiefungen auf, die mit Noppen oder Rippen (35) besetzt sind. Diese dienen einer verbesserten Griffigkeit. An einem Ende weist der Clip (6) eine Öse (36) auf, durch die ein Halteband gezogen und so befestigt werden kann.

Der Clip weist einen runden, pilzförmigen Hinterschnitt mit einem Zapfen auf. Der Zapfen rastet im Rastpunkt (39) ein.

Die Federplatte stützt sich auf einer Seite am Maskenwulst (3) ab. Beim Einführen des Clips (6) in den Rastpunkt wird die Federplatte gegen den Maskenwulst (3) gedrängt und verform diesem elastisch. Im Rastpunkt sichert der, in seine Ursprungsform zurück gefederte Wulst, die Position der Federplatte, die auch in seine Ursprungsposition zurück gefedert ist. Die Federplatte hält den Clip in der Rastposition im Rastpunkt. Zusätzlich sichert der Wulst, die Position der Federplatte.

Die Beweglichkeit des Clips relativ zum Maskenkörper ist durch den runden, pilzförmigem Hinterschnitt am Clip und die Fixierung im Trichter nur in einer Raumebene möglich. Dadurch ist der Clip im Wesentlichen nur in einer Ebene parallel zum Flügel bzw. parallel zum Wulst beweglich. Eine Beweglichkeit vom Clip hin zum Patientengesicht ist lediglich in einem Bereich von 1° bis 10° bevorzugt 1 - 7 °möglich.

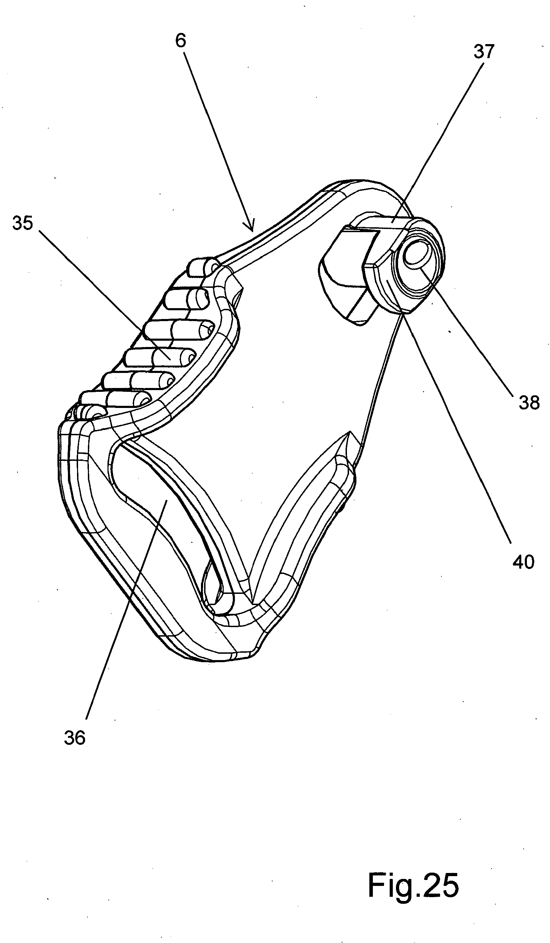

Fig. 25 zeigt eine perspektivische Darstellung des Bänderungsclips (6) von der im montierten Zustand zur Atemmaske hin orientierten Seite. Erkennbar sind ein Zapfen (37) mit pilzförmigem Hinterschnitt (40) . Der Hinterschnitt (40) kann umlaufend rund ausgebildet sein. Der Hinterschnitt (40) kann auch nur in Teilbereichen ausgebildet sein und beispielsweise zur Öffnung (36) ausgerichtet sein. Eine als Pfanne ausgeführte Vertiefung (38) im Kopf des Zapfens (37) sorgt im Zusammenspiel mit dem Rastzapfen (20) auf der Federplatte (18) des Maskenkörpers (1) für das Einrasten des Bänderungsclips (6) in der vorgesehenen Position am Flügel (12) des Maskenkörpers (1) . Im Kopf des Zapfens (37) kann auch ein Rastzapfen (20) ausgeführt sein, der mit einer als Pfanne ausgeführten Vertiefung (38)
auf der Federplatte (18) des Maskenkörpers (1) für das Einrasten des Bänderungsclips (6) sorgt.
Die Vertiefung (38) /der Rastzapfen (20) können in axialer Richtung des Zapfens (37) axial zentriert ausgeführt sein. Der Clip ist dann senkrecht nach oben und unten um die Achse des Zapfens (37) verschwenkbar.
Die Vertiefung (38) /der Rastzapfen (20) können auch in axialer Richtung des Zapfens (37) axial unzentriert ausgeführt sein. Eine Schwenkbewegung des Clips um die die Achse des Zapfens (37) führt dann zu einer Lösung der Verbindung zwischen Vertiefung (38) und Rastzapfen (20). Der Clip ist dann senkrecht nach oben und unten verschwenkbar, jedoch dient diese Bewegung in diesem Fall der Lösung des Clips aus der Rastposition.

## Patentansprüche

1. Atemmaske mit einem Maskenwulst (3), der die Maske zum Patienten hin abdichtet, einem Maskenkörper (1) und zumindest einem Flügel (12) der der Aufnahme eines Bänderungsclips (6) dient, wobei der Bänderungsclip eine Kopfbänderung an der Maske fixiert, wobei der Flügel (12) eine trichterförmige Aussparung (19) aufweist und der Clip (6) im Bereich eines Rastpunktes (39) der Aussparung verrastet **dadurch gekennzeichnet, dass** dem Rastpunkt (39) zugewandt an dem Maskenkörper (1) oder einer Federplatte (18) ein Rastzapfen (20) angeordnet ist, der bevorzugt eine gerundete Oberflächenkontur aufweist.

2. Atemmaske nach zumindest einem der vorhergehenden Ansprüche **dadurch gekennzeichnet, dass** die Aussparung (19) zwei unterschiedlich stark trichterförmig geformte Bereiche (19a, 19b) aufweist.

3. Atemmaske nach zumindest einem der vorhergehenden Ansprüche **dadurch gekennzeichnet, dass** der Rastpunkt (39) im engsten Bereich der trichterförmigen Aussparung (19) angeordnet ist.

4. Atemmaske nach zumindest einem der vorhergehenden Ansprüche **dadurch gekennzeichnet, dass** der Flügel (12) federnd ausgebildet ist.

5. Atemmaske nach zumindest einem der vorhergehenden Ansprüche **dadurch gekennzeichnet, dass** der Maskenkörper im Bereich des Flügels (12) eine Federplatte (18) aufweist, die benachbart zu der und/oder innerhalb der Aussparung (19) angeordnet ist.

6. Atemmaske nach zumindest einem der vorhergehenden Ansprüche **dadurch gekennzeichnet, dass** der Maskenkörper Leitstrukturen (13) aufweist, die den Patienten auf die Aussparung (19) und die Fügerichtung der Clips hinweisen, wobei die Leitstrukturen (13) an der Basis der Federplatte (18) angeordnet sind.

7. Atemmaske nach zumindest einem der vorhergehenden Ansprüche **dadurch gekennzeichnet, dass** der Trichter (19) den Clip (6) automatisch zum Rastpunkt (39) führt, in dem der Clip (6) durch die Federplatte (18) fixiert wird.

8. Atemmaske nach zumindest einem der vorhergehenden Ansprüche **dadurch gekennzeichnet, dass** der Clip (6) eine Vertiefung (38) aufweist, die im Zusammenspiel mit dem Rastzapfen (20) für das Einrasten des Clips (6) in der vorgesehenen Position am Flügel (12) in dem Rastpunkt (39) sorgt, wobei der Rastzapfen (20) an dem Maskenkörper oder der Federplatte angeordnet ist.

9. Atemmaske nach zumindest einem der vorhergehenden Ansprüche **dadurch gekennzeichnet, dass** der Clip (6) an seiner Ober- und Unterseite Vertiefungen oder Erhebungen aufweist, die mit Noppen oder Rippen (35) besetzt sind und an einem Ende eine Öse (36) aufweist, durch die ein Halteband gezogen und so befestigt werden kann.

10. Atemmaske nach zumindest einem der vorhergehenden Ansprüche **dadurch gekennzeichnet, dass** der Clip (6) einen Zapfen (37) mit pilzförmigem Hinterschnitt aufweist.

11. Atemmaske nach zumindest einem der vorhergehenden Ansprüche **dadurch gekennzeichnet, dass** der Clip (6) einen Zapfen (37) mit pilzförmigem Hinterschnitt aufweist sowie eine als Pfanne ausgeführte Vertiefung (38) im Kopf des Zapfens (37) aufweist.

12. Atemmaske nach zumindest einem der vorhergehenden Ansprüche **dadurch gekennzeichnet, dass** die Vertiefung (38) im Zusammenspiel mit dem Rastzapfen (20), auf der Federplatte (18), des Maskenkörpers (1) für das Einrasten des Bänderungsclips (6) in der vorgesehenen Position am Flügel (12) des Maskenkörpers (1) sorgt.

13. Atemmaske nach zumindest einem der vorhergehenden Ansprüche **dadurch gekennzeichnet, dass** die Vertiefung (38)/der Rastzapfen (20) in axialer Richtung des Zapfens (37) axial zentriert ausgeführt sind.

14. Atemmaske nach zumindest einem der vorhergehenden Ansprüche **dadurch gekennzeichnet, dass** die Vertiefung (38) /der Rastzapfen (20) in axialer Richtung des Zapfens (37) axial unzentriert ausgeführt sind, eine Schwenkbewegung des Clips um die Achse des Zapfens (37) zu einer Lösung der Verbindung zwischen Vertiefung (38) und Rastzapfen (20) führt.

## Claims

1. A breathing mask having a mask bead (3), which seals the mask toward the patient, a mask body (1) and at least one wing (12) which serves to receive a harness clip (6), wherein the harness clip fixes a head harness to the mask, wherein the wing (12) has a funnel-shaped recess (19) and the clip (6) catches in the region of a latching point (39) of the recess, **characterized in that** a latching pin (20) is arranged facing the latching point (39) on the mask body (1) or a spring plate (18), said latching pin preferably having a rounded surface contour.

2. The breathing mask according to at least one of the preceding claims, **characterized in that** the recess (19) has two regions (19a, 19b) which are funnel-shaped to differing degrees.

3. The breathing mask according to at least one of the preceding claims, **characterized in that** the latching point (39) is arranged in the narrowest region of the funnel-shaped recess (19) .

4. The breathing mask according to at least one of the preceding claims, **characterized in that** the wing (12) is configured elastically.

5. The breathing mask according to at least one of the preceding claims, **characterized in that** the mask body has a spring plate (18) in the region of the wing (12), which spring plate is arranged adjacent to and/or within the recess (19).

6. The breathing mask according to at least one of the preceding claims, **characterized in that** the mask body has guide structures (13) which indicate the recess (19) and the joining direction of the clips to the patient, wherein the guide structures (13) are arranged on the base of the spring plate (18).

7. The breathing mask according to at least one of the preceding claims, **characterized in that** the funnel (19) automatically guides the clip (6) to the latching point (39), in which the clip (6) is fixed by the spring plate (18) .

8. The breathing mask according to at least one of the preceding claims, **characterized in that** the clip (6) has a cavity (38) which ensures, in the interaction with the latching pin (20), that the clip (6) engages in the latching point (39) in the position provided on the wing (12), wherein the latching pin (20) is arranged on the mask body or the spring plate.

9. The breathing mask according to at least one of the preceding claims, **characterized in that** the clip (6) has on its upper and lower side cavities or elevations which are occupied by studs or ribs (35) and has, at one end, an eyelet (36), through which a securing strap can be pulled and fastened in this manner.

10. The breathing mask according to at least one of the preceding claims, **characterized in that** the clip (6) has a pin (37) having a mushroom-shaped undercut.

11. The breathing mask according to at least one of the preceding claims, **characterized in that** the clip (6) has a pin (37) having a mushroom-shaped undercut as well as a cavity (38) designed as a ladle in the head of the pin (37) .

12. The breathing mask according to at least one of the preceding claims, **characterized in that** the cavity (38), interacting with the latching pin (20), on the spring plate (18), of the mask body (1) ensures that the harness clip (6) engages in the position provided on the wing (12) of the mask body (1).

13. The breathing mask according to at least one of the preceding claims, **characterized in that** the cavity (38) of the latching pins (20) is designed to be axially centered in the axial direction of the pin (37).

14. The breathing mask according to at least one of the preceding claims, **characterized in that** the cavity (38) of the latching pins (20) is designed to be axially uncentered in the axial direction of the pin (37), a swiveling movement of the clip about the axis of the pin (37) leads to a loosening of the connection between the cavity (38) and the latching pins (20).

## Revendications

1. Masque respiratoire avec une jupe de masque (3) assurant l'étanchéité du masque par rapport au patient, un corps de masque (1) et au moins une aile (12) servant à recevoir une attache de harnais (6), dans lequel l'attache de harnais fixe un harnais de tête sur le masque, dans lequel l'aile (12) présente une encoche en forme d'entonnoir (19) et l'attache (6) s'encliquète dans la région d'un point d'encliquetage (39) de l'encoche, **caractérisé en ce qu'**un tenon d'encliquetage (20) tourné vers le point d'encliquetage (39) et présentant de préférence un contour de surface supérieure arrondi est disposé sur le corps de masque (1) ou sur une plaque élastique (18).

2. Masque respiratoire selon l'une au moins des revendications précédentes, **caractérisé en ce que** l'encoche (19) présente deux régions en forme d'entonnoir (19a, 19b) de différentes épaisseurs.

3. Masque respiratoire selon l'une au moins des revendications précédentes, **caractérisé en ce que** le point d'encliquetage (39) est disposé dans la région la plus étroite de l'encoche en forme d'entonnoir (19).

4. Masque respiratoire selon l'une au moins des revendications précédentes, **caractérisé en ce que** l'aile (12) est conçue de manière élastique.

5. Masque respiratoire selon l'une au moins des revendications précédentes, **caractérisé en ce que** le corps de masque présente une plaque élastique (18) dans la région de l'aile (12), laquelle est disposée à côté de l'encoche (19) et/ou dans celle-ci.

6. Masque respiratoire selon l'une au moins des revendications précédentes, **caractérisé en ce que** le corps de masque présente des structures de guidage (13) indiquant l'encoche (19) et la direction d'assemblage de l'attache aux patients, dans lequel les structures de guidage (13) sont disposées à la base de la plaque élastique (18).

7. Masque respiratoire selon l'une au moins des revendications précédentes, **caractérisé en ce que** l'entonnoir (19) amène automatiquement l'attache (6) vers le point d'encliquetage (39), au niveau duquel l'attache (6) est fixée par la plaque élastique (18).

8. Masque respiratoire selon l'une au moins des revendications précédentes, **caractérisé en ce que** l'attache (6) présente un renfoncement (38) assurant, en coopération avec le tenon d'encliquetage (20), l'encliquetage de l'attache (6) dans le point d'encliquetage (39) dans la position prévue sur l'aile (12), le tenon d'encliquetage (20) étant disposé sur le corps de masque ou sur la plaque élastique.

9. Masque respiratoire selon l'une au moins des revendications précédentes, **caractérisé en ce que** l'attache (6) présente des renfoncements ou des surélévations sur sa face supérieure et inférieure, lesquels sont pourvus de bossages ou de nervures (35) et présentent un œillet (36) à une extrémité, à travers lequel une bande de maintien peut être tirée et ainsi fixée.

10. Masque respiratoire selon l'une au moins des revendications précédentes, **caractérisé en ce que** l'attache (6) présente un tenon (37) avec une contre-dépouille en forme de champignon.

11. Masque respiratoire selon l'une au moins des revendications précédentes, **caractérisé en ce que** l'attache (6) présente un tenon (37) avec une contre-dépouille en forme de champignon, ainsi qu'un renfoncement (38) conçu comme une cuvette dans la tête du tenon (37).

12. Masque respiratoire selon l'une au moins des revendications précédentes, **caractérisé en ce que** le renfoncement (38), en coopération avec le tenon d'encliquetage (20), assure, sur la plaque élastique (18) du corps de masque (1), l'encliquetage de l'attache de harnais (6) dans la position prévue sur l'aile (12) du corps de masque (1) .

13. Masque respiratoire selon l'une au moins des revendications précédentes, **caractérisé en ce que** le renfoncement (38) des tenons d'encliquetage (20) est centrée axialement dans la direction axiale du tenon (37).

14. Masque respiratoire selon l'une au moins des revendications précédentes, **caractérisé en ce que** le renfoncement (38) des tenons d'encliquetage (20) n'est pas centré axialement dans la direction axiale du tenon (37) , un mouvement de pivotement de l'attache autour de l'axe du tenon (37) entraîne un détachement de l'assemblage entre le renfoncement (38) et les tenons d'encliquetage (20) .
